# EUROPEAN PATENT SPECIFICATION

(11) **EP 4 169 484 B1**
(45) Date of publication and mention of the grant of the patent: **10.12.2025**
(21) Application number: 22202067.9
(22) Date of filing: 18.10.2022
(51) Int. Cl.: A61F 2/20, A61M 16/04

(54) **VOICE PROSTHESIS WITH UMBRELLA VALVE**
STIMMPROTHESE MIT SCHIRMVENTIL
PROTHÈSE VOCALE AVEC VALVE DE PARAPLUIE

(30) Priority: 19.10.2021 US 202163257324 P; 19.10.2021 US 202163257334 P; 12.10.2022 US 202217964392
(43) Date of publication of application: 26.04.2023
(73) Proprietor: Freudenberg Medical, LLC, Carpinteria, CA 93013 (US)
(72) Inventor: Glen, Kevin Alan, Ventura, 93003 (US); Konda, Manoj Reddy, Santa Clara, 95051 (US); Kraemer, Griffin Malone, Santa Barbara, 93101 (US)
(74) Representative: Schaumburg und Partner Patentanwälte mbB

(56) References cited:
- EP-A1- 0 078 685
- WO-A1-2020/148297
- US-A1- 2005 256 573

## Description

### FIELD

The present disclosure relates to a voice prosthesis with an umbrella valve. The present disclosure relates to a voice prosthesis with a floating disc valve.

### BACKGROUND

This section provides background information related to the present disclosure which is not necessarily prior art.

Voice prostheses are inserted into a fistula in a tracheoesophageal wall to allow a laryngectomized patient to produce speech by pushing air from their lungs through the valve and up into their mouth. The typical voice prosthesis can include a flap valve that can develop a biological growth that can foul the hinge of the flap valve.

Accordingly, it is desirable to provide a new style of valve that can better function with biofilm and ingrowth and provide a simple assembly method with improved manufacturing.

Document WO2020/148297A1 discloses a speaking valve for use with a tracheostomy and voice prosthesis having a valve element movable by finger pressure between a first position in which the valve allows air to pass through it and a second position in which the passage of air is substantially resisted or prevented. A biasing element such as foam or a spring is provided to bias the valve element towards the first position when no finger pressure is applied. The valve element is arranged such that expiration through the valve at a rate in excess of predetermined rate causes it to move into a third position in which air resistance through the valve is less than when the valve element is in its first position. This allows for air release if e.g., a user should cough.

Document EP0078685 A1 discloses an externally worn tracheal valve has a valve assembly supporting a flexible, resilient lightweight diaphragm centrally thereof. The diaphragm is folded towards the trachea so that air expelled from the trachea will tend to unfold the diaphragm to an extended position to close the valve. During normal breathing, the diaphragm remains at least partially folded and the valve remains open. During voice exhalation, the diaphragm extends to close the valve.

### SUMMARY

This section provides a general summary of the disclosure, and is not a comprehensive disclosure of its full scope or all of its features.

The invention is defined in claim 1.

A voice prosthesis is provided for insertion into a fistula opening in a tracheoesophageal wall. The voice prosthesis includes a tubular elastomeric body having a central passage therein and having a valve seat on one end thereof. A pair of flanges each extending radially outwardly from opposite ends of the tubular elastomeric body. An umbrella shaped valve is disposed in at least a portion of the tubular elastomeric body, the umbrella shaped valve engaging the valve seat in a normal state and the umbrella shaped valve being inverted in an open actuated state.

According to another aspect of the present disclosure, a voice prosthesis is provided for insertion into a fistula opening in a tracheoesophageal wall. The voice prosthesis includes a tubular elastomeric body having a central passage therein and having a valve seat on one end thereof. A pair of flanges each extend radially outwardly from opposite ends of the tubular elastomeric body. A floating valve is supported by a flexible web. The floating valve engages the valve seat in a normal state and the floating valve is movable to an open position in an actuated state wherein the flexible web flexes to allow the movement of the floating valve.

According to a further aspect of the present disclosure, a voice prosthesis is provided for insertion into a fistula opening in a tracheoesophageal wall. The voice prosthesis includes a tubular elastomeric body having a central passage therein and having a valve seat on one end thereof. A pair of flanges each extending radially outwardly from opposite ends of the tubular elastomeric body. An umbrella shaped valve is disposed in at least a portion of the tubular elastomeric body, the umbrella shaped valve engaging the valve seat in a normal state and the umbrella shaped valve flexing upward in an open actuated state. The umbrella shaped valve can further be supported by a flexible web. The floating umbrella shaped valve engages the valve seat in a normal state and is movable to an open position in an actuated state wherein the flexible web flexes to allow movement of the valve.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is a partial cut-away perspective view of a voice prosthesis with an umbrella valve according to the principles of the present disclosure;
FIG. 2 is a cross-sectional view of a voice prosthesis with an umbrella valve in a closed natural state;
FIG. 3 is a cross-sectional view of the voice prosthesis with an umbrella valve in an actuated open state;
FIG. 4 is a plan view of the voice prosthesis with the umbrella valve removed for illustrative purposes;
FIG. 5 is a partial cut-away perspective view of a voice prosthesis with a floating valve according to the principles of the present disclosure;
FIG. 6 is a cross-sectional view of a voice prosthesis with a floating valve in a closed natural state;
FIG. 7 is a cross-sectional view of the voice prosthesis with a floating valve in an actuated open state;
FIG. 8 is a plan view of the voice prosthesis with the floating valve removed for illustrative purposes;
FIG. 9 is a partial cut-away perspective view of a voice prosthesis with a floating umbrella valve according to the principles of the present disclosure;
FIG. 10 is a cross-sectional view of a voice prosthesis with a floating umbrella valve in a closed natural state;
FIG. 11 is a cross-sectional view of the voice prosthesis with a floating umbrella valve in an actuated open state; and
FIG. 12 is a plan view of the voice prosthesis with the floating umbrella valve removed for illustrative purposes.

Corresponding reference numerals indicate corresponding parts throughout the several views of the drawings.

### DETAILED DESCRIPTION

Example embodiments will now be described more fully with reference to the accompanying drawings.

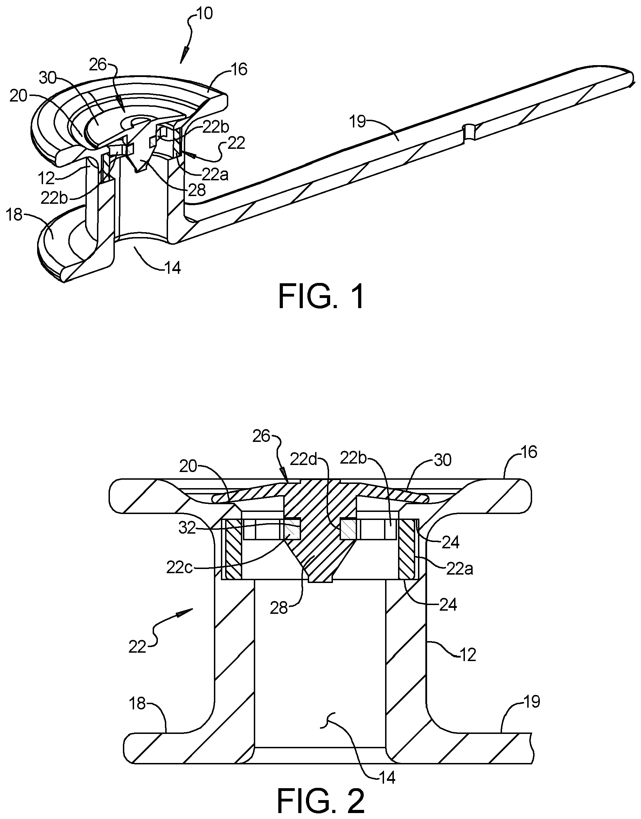

With reference to FIGS. 1-4, a voice prosthesis 10 is shown for insertion into a fistula in a tracheoesophageal wall. The voice prosthesis 10 includes a tubular elastomeric body 12 having a central passage 14 therein. A pair of flanges 16, 18 extend radially outwardly from opposite ends of the tubular elastomeric body 12. A strap 19 can extend from the flange 18. A valve seat 20 is provided on one end of the tubular elastomeric body 12.

A rigid valve support 22 is disposed within the central passage 14. The valve support 22 can include an annular body 22a and a plurality of radially inwardly extending arms 22b that terminate at a central hub 22c having an aperture 22d therein. The rigid valve support 22 can be supported within the central passage 14 between a pair of shoulders 24 or by an adhesive or other known techniques.

An umbrella shaped valve 26 is supported by the valve support 22 in at least a portion of the tubular elastomeric body 12. The umbrella shaped valve 26 includes a stem 28 and a generally round canopy 30 that can have a parabolic or cone shaped cross-section in a normal, closed state with the edges of the canopy 30 contacting the valve seat 20. The stem 28 can include a recessed groove 32 that receives the central hub 22c therein.

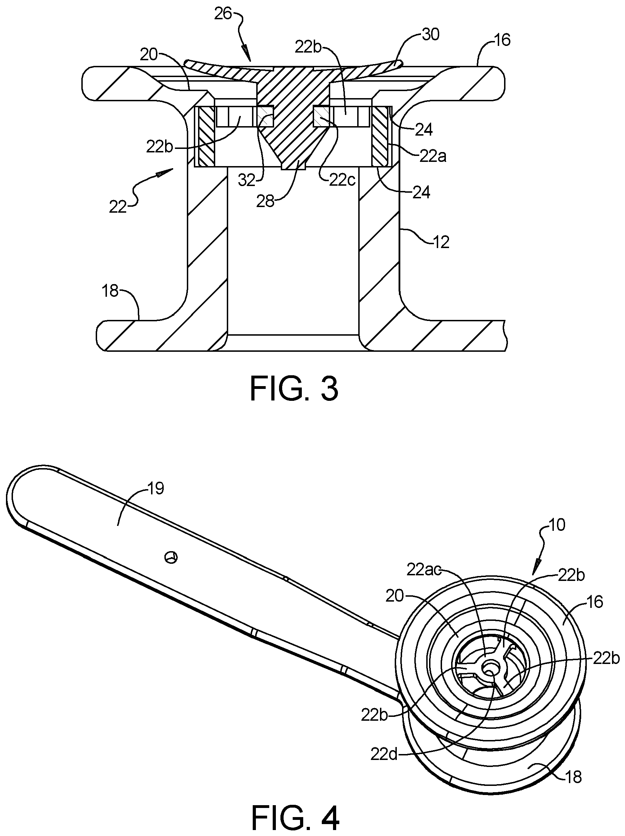

The umbrella shaped valve 26 is made from an elastomeric material such that in an actuated state during speech, the canopy 30 can be flexed upward into an open position, as shown in FIG. 3. The upward flexing of the canopy 30 can be similar to the inversion of an umbrella under heavy winds.

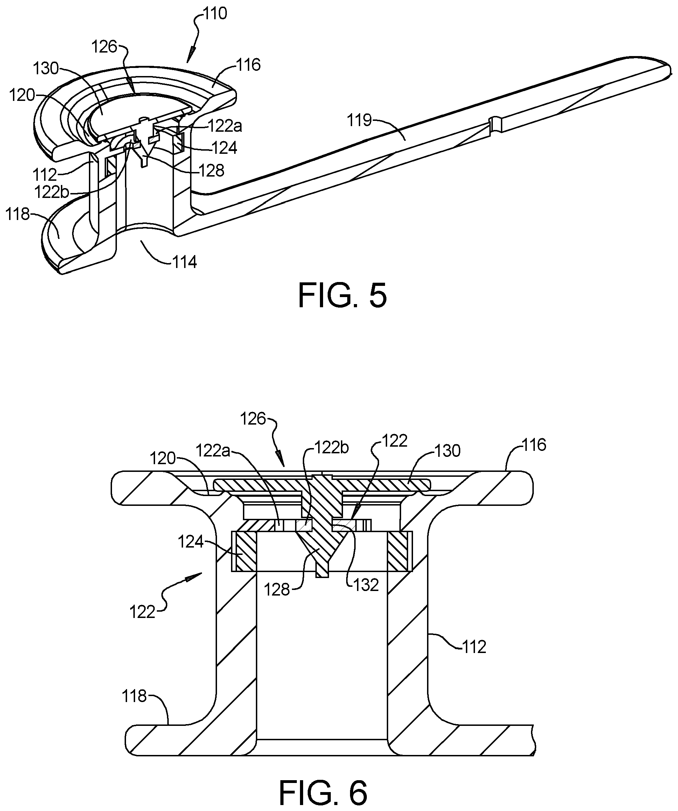

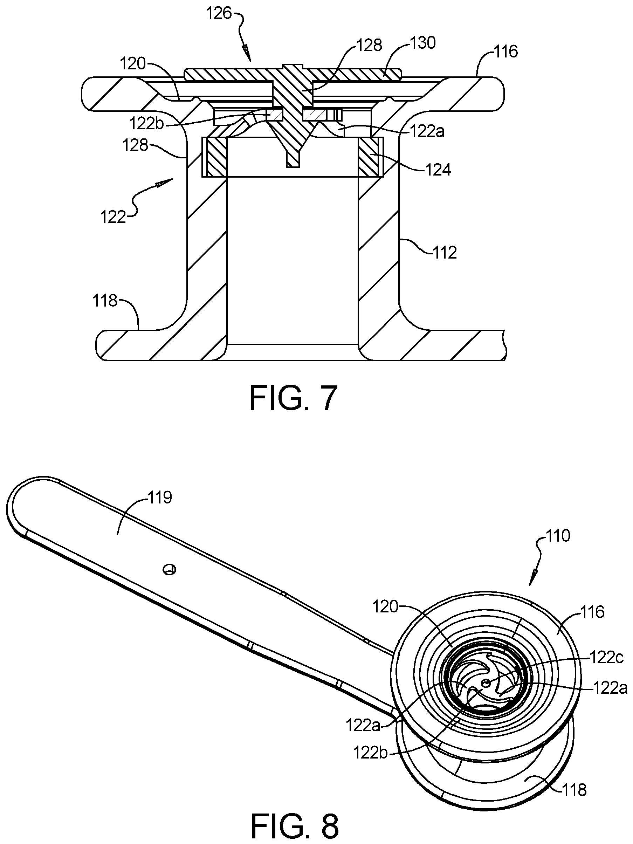

With reference to FIGS. 5-8, a voice prosthesis 110 is shown for insertion into a fistula opening in a tracheoesophageal wall. The voice prosthesis 110 includes a tubular elastomeric body 112 having a central passage 114 therein. A pair of flanges 116, 118 extend radially outwardly from opposite ends of the tubular elastomeric body 112. A strap 119 can extend from the flange 118. A valve seat 120 is provided on one end of the tubular elastomeric body 112.

A flexible valve support 122 is disposed within the central passage 114. As best shown in FIG. 5, the valve support 122 can include a flexible web including a plurality of radially inwardly extending flexible arms 122a that terminate at a central hub 122b having an aperture 122c therein. The flexible arms 122a can be spiral in shape to allow upward flexibility. The valve support 122 can be disposed within the central passage 114 and can be formed integral or separate from the elastomeric body 112. A rigid support ring 124 can be disposed within the elastomeric body 112.

A floating valve 126 is supported by the valve support 122 in at least a portion of the tubular elastomeric body 112. The floating valve 126 can include a stem 128 and a valve body 130 that can be a generally round disc. In a normal, closed state the valve body 130 contacts the valve seat 120. The floating valve 126 can be made from a rigid material such that in an actuated state during speech, the floating valve 126 stretches the flexible arms 122a so that the valve is opened. The stem 128 can include a recessed groove 132 that receives the central hub 122b therein. The floating valve 126 can alternatively be made from an elastomeric material to allow the valve body 130 to also flex toward an open position.

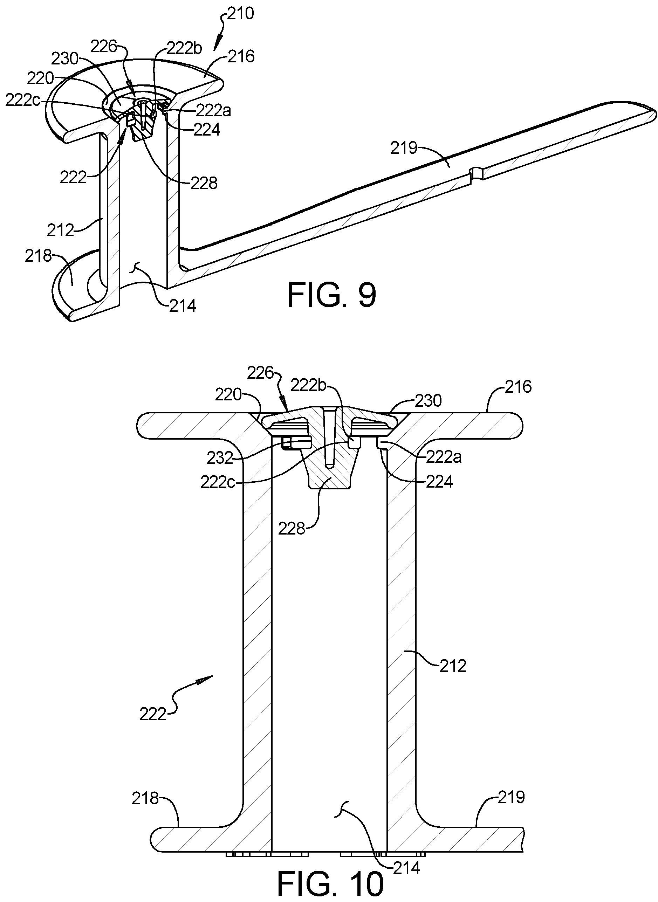

With reference to FIGS. 9-12, a voice prosthesis 210 is shown for insertion into a fistula opening in a tracheoesophageal wall. The voice prosthesis 210 includes a tubular elastomeric body 212 having a central passage 214 therein. A pair of flanges 216, 218 extend radially outwardly from opposite ends of the tubular elastomeric body 212. A strap 219 can extend from the flange 218. A valve seat 220 is provided on one end of the tubular elastomeric body 212.

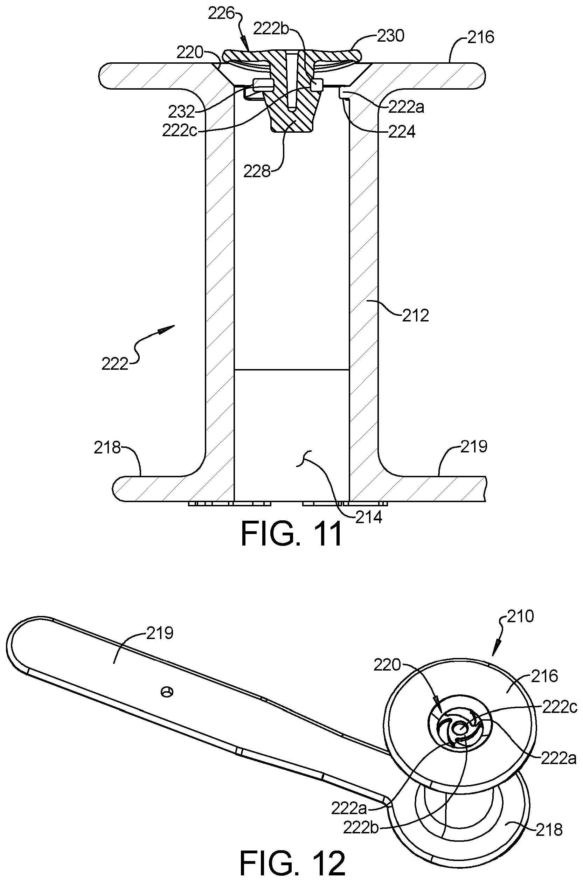

A flexible valve support 222 is disposed within the central passage 214. As best shown in FIG. 12, the valve support 222 can include a flexible web including a plurality of radially inwardly extending flexible arms 222a that terminate at a central hub 222b having an aperture 222c therein. The flexible arms 222a can be spiral in shape to allow upward flexibility. The valve support 222 can be disposed within the central passage 214 and can be formed integral or separate from the elastomeric body 212. A rigid support ring 224 can be disposed within the elastomeric body 212.

A floating umbrella valve 226 is supported by the valve support 222 in at least a portion of the tubular elastomeric body 212. The floating umbrella valve 226 includes a stem 228 and a generally round canopy 230 that can have a parabolic or cone shaped cross-section in a normal, closed state with the edges of the canopy 230 contacting the valve seat 220 The umbrella shaped valve 226 is made from an elastomeric material such that in an actuated state during speech, the canopy 230 can be flexed upward into an open position, as shown in FIG. 11. The upward flexing of the canopy 230 can be similar to the inversion of an umbrella under heavy winds. The stem 228 can include a recessed groove 232 that receives the central hub 222b therein.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

## Claims

1. A voice prosthesis for insertion into a fistula opening in a tracheoesophageal wall, comprising:
a tubular elastomeric body (112) having a central passage (112) therein and having a valve seat (120) on one end thereof;
a pair of flanges (116, 118) each extending radially outwardly from opposite ends of the tubular elastomeric body (112)
a valve includes a round valve body (130) and a stem (128) integrally formed with the round valve body (130) and extending from a center of the round valve body (130), **characterized by** the stem (128) being supported in an aperture (122c) of a central hub (122b) of a flexible support having a plurality of flexible arms (122a) that connect the central hub (122b) to the tubular elastomeric body (112) the valve body (130) engaging the valve seat (120) in a first state and the flexible support allowing the valve body (120) to be lifted away from the valve seat (120) in a second state.

2. The voice prosthesis according to claim 1, wherein the flexible support is made from an elastomeric material.

3. The voice prosthesis according to claim 1, wherein the floating valve is made from a rigid material.

4. The voice prosthesis according to claim 1, wherein the floating valve is made from an elastomeric material.

5. The voice prosthesis according to claim 4, wherein the floating valve is umbrella shaped wherein the round valve body (130) has one of a parabolic and cone shaped cross-section that is allowed to flex toward an open position in the second state.

6. The voice prothesis according to claim 1, wherein the flexible arms (122a) are spiral shaped.

## Patentansprüche

1. Stimmprothese zum Einführen in eine Fistelöffnung in einer tracheo-ösophagealen Wand, umfassend:
einen rohrförmigen Elastomerkörper (112) mit einem zentralen Durchgang (112) darin und mit einem Ventilsitz (120) an einem Ende davon;
ein Paar von Flanschen (116, 118), die sich jeweils von gegenüberliegenden Enden des rohrförmigen Elastomerkörpers (112) radial nach außen erstrecken,
ein Ventil, das einen runden Ventilkörper (130) und einen Schaft (128), der integral mit dem runden Ventilkörper (130) gebildet ist und sich von einer Mitte des runden Ventilkörpers (130) erstreckt, aufweist, **dadurch gekennzeichnet, dass** der Schaft (128) in einer Öffnung (122c) einer zentralen Nabe (122b) eines flexiblen Trägers mit einer Vielzahl von flexiblen Armen (122a), die die zentrale Nabe (122b) mit dem rohrförmigen Elastomerkörper (112) verbinden, getragen wird,
wobei der Ventilkörper (130) in einem ersten Zustand mit dem Ventilsitz (120) in Eingriff steht und der flexible Träger erlaubt, dass der Ventilkörper (120) in einem zweiten Zustand von dem Ventilsitz (120) weg gehoben wird.

2. Stimmprothese nach Anspruch 1, wobei der flexible Träger aus einem elastomeren Material besteht.

3. Stimmprothese nach Anspruch 1, wobei das schwimmende Ventil aus einem starren Material besteht.

4. Stimmprothese nach Anspruch 1, wobei das schwimmende Ventil aus einem elastomeren Material besteht.

5. Stimmprothese nach Anspruch 4, wobei das schwimmende Ventil schirmförmig ist, wobei der runde Ventilkörper (130) einen von einem parabolischen oder einem kegelförmigen Querschnitt aufweist, der sich in dem zweiten Zustand in Richtung zu einer offenen Position biegen kann.

6. Sprachprothese nach Anspruch 1, wobei die flexiblen Arme (122a) spiralförmig sind.

## Revendications

1. Prothèse vocale destinée à être insérée dans une ouverture de fistule dans une paroi trachéo-œsophagienne, comprenant :
un corps élastomère tubulaire (112) présentant un passage central (12) en son sein et présentant un siège de valve (120) à une extrémité de celui-ci ;
une paire de brides (116, 118) s'étendant chacune radialement vers l'extérieur à partir d'extrémités opposées du corps élastomère tubulaire (112) ;
une valve inclut un corps de valve rond (130) et une tige (128) formée d'un seul tenant avec le corps de valve rond (130) et s'étendant à partir d'un centre du corps de valve rond (130), **caractérisée en ce que** la tige (128) est supportée dans une ouverture (122c) d'un moyeu central (122b) d'un support flexible présentant une pluralité de bras flexibles (122a) qui relient le moyeu central (122b) au corps élastomère tubulaire (112), le corps de valve (130) venant en prise avec le siège de valve (120) dans un premier état et le support flexible permettant au corps de valve (120) d'être soulevé à l'écart du siège de valve (120) dans un second état.

2. Prothèse vocale selon la revendication 1, dans laquelle le support flexible est réalisé en matériau élastomère.

3. Prothèse vocale selon la revendication 1, dans laquelle la valve flottante est réalisée en un matériau rigide.

4. Prothèse vocale selon la revendication 1, dans laquelle la valve flottante est réalisée en matériau élastomère.

5. Prothèse vocale selon la revendication 4, dans laquelle la valve flottante présente une forme de parapluie dans laquelle le corps de valve rond (130) présente une section transversale parabolique ou conique qui peut fléchir vers une position ouverte dans le second état.

6. Prothèse vocale selon la revendication 1, dans laquelle les bras flexibles (122a) sont en forme de spirale.
